(19) **Europäisches Patentamt / European Patent Office / Office européen des brevets**

(11) **EP 4 715 730 A1**

(12) **EUROPEAN PATENT APPLICATION**

(43) Date of publication:
25.03.2026 Bulletin 2026/13

(21) Application number: 24201811.7

(22) Date of filing: 23.09.2024

(51) International Patent Classification (IPC):
*G06T 7/00* (2017.01)     *G06T 7/11* (2017.01)

(52) Cooperative Patent Classification (CPC):
**G06T 7/0012; G06T 7/11;** G06T 2207/10081;
G06T 2207/20081; G06T 2207/20084;
G06T 2207/30061; G06T 2207/30104

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: Siemens Healthineers AG
91301 Forchheim (DE)

(72) Inventors:
• **Sühling, Michael**
**91052 Erlangen (DE)**
• **Jürgens, Markus**
**91325 Adelsdorf (DE)**
• **Grasruck, Michael**
**90542 Eckental Forth (DE)**

(74) Representative: **Siemens Healthineers
Patent Attorneys
Postfach 22 16 34
80506 München (DE)**

(54) **CHARACTERIZATION OF A PERFUSION DEFECT**

(57)     For imaging-based characterization of a perfusion defect in a vessel structure for blood supply of an organ (5, 8) of a patient, medical imaging data (11, 12, 17) is received, wherein the medical imaging data (11, 12, 17) comprises energy resolved CT imaging data (11, 12, 17). A blood stream obstructing object (7) in the vessel structure is detected based on the medical imaging data (11, 12, 17). A perfusion defect score (16) for a target region (6a, 6b) of the at least one organ (5, 8), whose blood perfusion is potentially affected by the obstructing object (7), is determined depending on the energy resolved CT imaging data (11, 12, 17).

FIG 3

**Description**

The present invention is directed to a computer-implemented method for imaging-based characterization of a perfusion defect in a vessel structure for blood supply of at least one organ of a patient, a corresponding data processing system and a corresponding computer program product.

The present invention is in the field of medical imaging analysis, which is used for of creating data based representations, in particular visual representations, of the interior of a body based on the detection of physical signals and imaging data-based analysis of the resultant image data to support analysis, in particular visual representation, of the function of organs or tissues.

A pulmonary embolism is a potentially life-threatening condition that may occur when a blood clot, also denoted as thrombus, forms in one of the pulmonary arteries. The clot can block or partially obstruct the blood flow to the lungs, leading to various symptoms and complications. In medical imaging data, for example computed tomography, CT, imaging data, a pulmonary embolism may for example appear as a filling defect or an area of decreased contrast enhancement within the pulmonary arteries, for example.

Algorithms based on machine learning models, MLMs, to automatically detect pulmonary artery blood clots from medical imaging data are known. For example, the publication P. A. Grenier et al.: "Deep Learning-Based Algorithm for Automatic Detection of Pulmonary Embolism in Chest CT Angiograms", Diagnostics (Basel), 2023, 13(7) uses artificial neural networks, ANNs, to detect such clots in CT angiography, CTA, imaging data.

However, merely detecting the presence and position of the blood clot does not necessarily allow to reliably estimate the criticality of resulting perfusion defects.

The publication E. A. Boyden: "Segmental Anatomy of the Lungs. A Study of the Patterns of the Segmental Bronchi and Related Pulmonary Vessels", The Blakiston Division, McGraw-Hill Book Company, Inc., New York, 1955, describes a hierarchical classification of bronchi.

The publication H. Koike et. al.: "Quantification of lung perfusion blood volume (lung PBV) by dual-energy CT in patients with chronic thromboembolic pulmonary hypertension (CTEPH) before and after balloon pulmonary angioplasty (BPA): Preliminary results.", Eur. J. Radiol., 2016, 85(9),1607, describes how the lung perfusion blood volume, PBV, may be quantified by computing the ratio of the lung PBV to the pulmonary artery enhancement in dual energy CT, DECT, imaging data.

In the publication by O. Ronneberger et al.: "U-Net: Convolutional Networks for Biomedical Image Segmentation" (arXiv: 1505.04597), the U-Net architecture is described, a widely used CNN architecture for image segmentation, which may, however, also be used for other computer vision tasks, in particular image-to-image tasks.

It is an objective of the present invention to provide a possibility to automatically characterize a perfusion defect in our vessel structure for blood supply of at least one organ of a patient.

This objective is achieved by the subject matter of the independent claim. Further implementations and preferred embodiments are subject matter of the dependent claims.

The invention is based on the idea to not only detect a bloodstream obstructing object in a vessel structure for blood supply of at least one organ of a patient, but also to determine a perfusion defect score for a target region, which is potentially affected by the obstructing object, depending on energy resolved CT imaging data.

According to an aspect of the invention, a computer-implemented method for imaging-based characterization of a perfusion defect in a vessel structure, in particular a blood vessel structure, for blood supply of at least one organ of a patient is provided. Therein, medical imaging data depicting the at least one organ and the vessel structure is received, for example from a medical imaging device or from a data storage device. The medical imaging data comprises or consists of energy resolved CT imaging data. A blood stream obstructing object, for example a blood clot, in the vessel structure is detected based on the medical imaging data. A perfusion defect score for a target region of the at least one organ, wherein the blood perfusion in the target region is potentially affected by the obstructing object, is determined depending on the energy resolved CT imaging data.

Unless stated otherwise, all steps of the computer-implemented method may be performed by a data processing system, which comprises at least one data processing device. In particular, the at least one data processing device is configured or adapted to perform the steps of the computer-implemented method. For this purpose, the at least one data processing device may for example store a computer program comprising instructions which, when executed by the at least one data processing device, cause the at least one data processing device to execute the computer-implemented method. The expressions "data processing system" and "at least one data processing device" may be used interchangeably, here and in the following. This holds also for respective expressions derived therefrom. In other words, claims for the system according to the invention can be improved with features described or claimed in the context of the methods and vice versa. In this case, the functional features of the method are embodied by objective units or modules of the system.

In case the at least one data processing device comprises two or more data processing devices, certain steps carried out by the at least one data processing device may also be understood such that different data processing devices carry out different steps or different parts of a step. In particular, it is not required that each data processing device carries out the steps completely. In other words, carrying out the steps may be distributed amongst the two or more data processing devices.

From each implementation of the computer-implemented method, a respective implementation of a method for imaging-based characterization of a perfusion defect in a vessel structure for blood supply of at least one organ of a patient, which is not purely computer-implemented, is obtained by including respective steps of generating the medical imaging data, for example by the medical imaging device.

The energy resolved CT imaging data may comprise contrast enhanced imaging data obtained with X-ray computed tomography (CT) using contrast agents. Contrast agents for X-ray CT are often iodine-based contrast agents. This is useful to highlight structures such as blood vessels that otherwise would be difficult to delineate from their surroundings. Using contrast material can also help to obtain functional information about tissues. Often, images are taken both with and without radiocontrast.

The energy resolved CT imaging data, also denoted as spectrally resolved CT imaging data, is denoted as such, since it comprises imaging data for different energies of the detected X-rays or X-ray quanta, respectively. This may for example be achieved by using different X-rays spectra, for example by using different X-ray filters, by using X-ray sources to provide X-rays with different energy spectra, such as in dual source CT scanners or by kV-switching of a single X-ray source, or by using energy-resolving X-ray detectors, in particular by means of photon counting CT, PCCT, where different energy thresholds may be used for counting the incident photons. The medical imaging data, for example the energy resolved CT imaging data, may comprise image data derived from energy resolved CT imaging data raw data such as one or more reconstructed CT image volumes, including for example a regular CT image volume, imaging data with material decomposition information, virtual monoenergetic imaging data, an iodine-enhanced CT image volume, virtual non-contrast imaging data, a dual-energy ratio (DER), CT image volume DER, and so forth.

The energy resolved CT imaging data allows to differentiate between different materials. This may for example be exploited by computing respective contrast agent maps, for example iodine maps, or dual energy ratio, DER maps, which may then be analyzed to determine the perfusion defect score.

Detecting the obstructing object comprises, in particular, determining the location of the obstructing object in the vessel structure or a region of the vessel structure, wherein the obstructing object is located. This information is, for example, used to identify the target region. The detection of the obstructing object may be done depending on the energy resolved CT imaging data as well. Alternatively, the medical imaging data may comprise further imaging data and the obstructing object is detected depending on the further imaging data. For detecting the obstructing object, for example a known image analysis algorithm may be used.

If not stated otherwise, here and in the following, CT imaging data may for example comprise one or more three-dimensional CT reconstructions, also denoted as reconstructed CT image volumes.

The at least one organ may for example comprise the lungs of the patient or a part of the lungs including, in particular, a bronchial tree of the lungs and/or parenchymal tissue of the lungs. The at least one organ may for example also include the vessel structure. The vessel structure may for example comprise a structure or tree of pulmonary arteries of the lungs.

The perfusion defect score may be a final result of the computer-implemented method according to the invention. In particular, by determining the perfusion defect score, the perfusion defect in a vessel structure is characterized. Depending on the perfusion defect score, for example the severity or criticality of the obstructing object's effect may be evaluated.

The size and location of the obstructing object can vary, ranging from small and peripheral to large and central. Consequently, the effect of the obstructing object can be very minor but can also be significant and even life-threatening, since the obstructing object may reduce or completely cut off blood supply to the downstream lung tissue, leading to decreased lung perfusion. The perfusion defect score may therefore be used to differentiate between more or less severe obstructing objects.

In particular, the perfusion defect score may be regarded as being assigned or associated with the concrete obstructing object. In other words, determining the perfusion defect score may be regarded as classifying the obstructing object according to the severity of its effect on the perfusion of the at least one organ.

In some implementations, the described steps of the computer-implemented method according to the invention may be carried out twice or multiple times for different obstructing objects at different locations, respectively. In this case, the different obstructing objects maybe ranked and/or filtered according to their perfusion defect scores.

According to several embodiments, a segmentation dividing the at least one organ into a plurality of segments is generated based on the medical imaging data, wherein the plurality of segments is hierarchically classified according to their blood supply by the vessel structure. The target region is determined to comprise or consist of one or more target segments of the plurality of segments, whose blood perfusion is potentially affected by the obstructing object according to the hierarchical classification.

In such embodiments, it is exploited that the anatomy of human organs, in particular the lungs, is well-known and so is the relation between segments of the organs and the respective vessels supplying them with blood. Thus, by using the segmentation including the hierarchical classification, a particularly reliable characterization of the perfusion defect is achieved.

For example, a known algorithm for medical image segmentation may be used to generate the plurality of segments.

In case the at least one organ comprises the lungs, the segmentation may for example be generated according to a bronchopulmonary segment model or according to a lung segmentation model, for example on the basis of the hierarchical classification according to Boyden mentioned in the introductory part of the present disclosure. The hierarchical classification of the plurality of segments according to their blood supply by the vessel structure may for example be understood such that it is defined for each segment of the plurality of segments, whether blood supply of the respective segment by the vessel structure depends on the blood supply of one or more other segments of the plurality of segments via the vessel structure and, if applicable, which other segments of the plurality of segments. In other words, given a segment of the plurality of segments and assuming that its blood supply via the vessel structure is completely blocked due to an obstructing object in the respective part of the vessel structure, it is defined to which other segments of the plurality of segments blood supply via the vessel structure would therefore also be cut off.

For example, by detecting the obstructing object, a root segment of the plurality of segments may be identified, which is the segment of the plurality of segments, where the obstructing object is located in the respective part of the vessel structure, which directly supplies the respective segment. In other words, the root segment is the segment of the plurality of segments, whose blood supply would be affected by the obstructing object, even if the blood supply of no other segment, in particular a hierarchically higher ordered segment, would be affected by the obstructing object. The root segment may also be determined as the segment of the plurality of segments to which the obstructing object lies closest.

The one or more target segments comprise the root segment. In some cases, the one or more target segments consist of the root segment. Alternatively, the one or more target segments comprises one or more downstream segments of the plurality of segments. In particular, the one or more target segments consist of the root segment and the one or more downstream segments. The one or more downstream segments may be those segments, whose blood supply via the vessel structure would be cut off in case the blood supply of the root segment was cut off.

According to several embodiments, for each of the one or more target segments, a respective segment perfusion defect score is determined based on the energy resolved CT imaging data and the perfusion defect score is determined depending on the segment perfusion defect scores, in particular depending on all segment perfusion defect scores of all target segments.

By evaluating the effect of the obstructing object individually for each target segment, a comprehensive analysis of the total perfusion defect is achieved and therefore a particularly reliable characterization of the perfusion defect.

For example, the perfusion defect score may be computed depending on or as a sum of the segment perfusion defect scores of the target segments or an average of the segment perfusion defect scores of the target segments or a maximum segment perfusion defect score of the target segments, et cetera. The perfusion defect score may also be computed as the number of target segments, whose segment perfusion defect score is at least a predefined threshold value.

According to several embodiments, the perfusion blood volume, PBV, may be determined for each of the target segments depending on the energy resolved CT imaging data and the segment perfusion defect scores may be computed depending on the respective PBV.

The PBV allows to reliably quantify the blood perfusion in the target segments and therefore to reliably quantify the effect of the obstructing object on the blood perfusion in the target segments. In particular, the PBV may be determined based on the quantification of iodine uptake of a contrast agent, for example iodine, by the organ, in particular lung parenchyma, based on the energy resolved CT imaging data. For example, the lower the PBV is and/or the larger the region of reduced PBV is, the larger is the effect of the obstructing object on the blood perfusion of the respective target segment and, consequently, the larger is the respective segment perfusion defect score, for example.

According to several embodiments, for each of the one or more target segments, a size of a perfusion defect region, for example a volume of the perfusion defect region, in the respective target segment is determined based on the energy resolved CT imaging data. The respective segment perfusion defect score is determined depending on or as the size of the perfusion defect region.

The size of the perfusion defect region may for example be determined as a volume in the respective target segment, where the PBV is less than a predefined threshold, for example.

The size of the perfusion defect region represents a reliable measure for assessing the effect of the obstructing object on the blood perfusion of the respective target segment. Thus, the segment perfusion defect scores and, consequently, perfusion defect score, may be determined in a particularly reliable way.

For example, the perfusion defect score may be computed as a total perfusion defect size or, in other words, total perfusion defect volume, which is given by a sum of the sizes of the perfusion defect regions of the individual target segments. The perfusion defect score may also be computed as a ratio of the total perfusion defect size to a predetermined or predefined total lung volume of the patient.

According to several embodiments, at least one PBV value for the target region is determined depending on the energy resolved CT imaging data and the perfusion defect score is determined depending on the at least one PBV value.

The at least one PBV value may for example be a single PBV value for the whole target region or a respective segment PBV value for each target segment.

A PBV value may be a value derived from the PBV of the target region or the respective target segment. For example, the at least one PBV value may correspond to the total perfusion defect size or the at least one PBV value comprises a respective segment PBV value for each of the one or more target segments, for example the respective size of the perfusion defect region of the respective target segment.

According to several embodiments, the medical imaging data, in particular the energy resolved CT imaging data, comprises photon-counting CT, PCCT, imaging data and the obstructing object is detected based on the PCCT imaging data.

Such embodiments are particularly beneficial, since on the one hand, PCCT allows for ultra-high resolution CT reconstructions and, on the other hand, also naturally may provide the data in energy resolved form by evaluating different energy thresholds when counting the incident X-ray photons. Thus, the obstructing object may be detected in a particularly accurate way and also the perfusion defect score may be obtained in a particularly reliable way.

Photon-counting detectors for computed tomography enable the generation of spectrally resolved computed tomography medical image data. A photon-counting detector can be configured to acquire x-ray projection data in a plurality on energy bins. These window parameters of the energy bins are defined by energy thresholds corresponding to the (spectral) energy of the X-ray photons to be detected. The energy thresholds of the detector can be predetermined and set by a respective control logic which can be implemented in the photon counting detector. A plurality of energy bins, for example four, can be used to cover the range of energies of the acquired x-ray projection data.

The window parameters of the energy bins may be pre-defined before the start of the acquisition of the energy resolved imaging raw data. To improve the quality of the acquired photon-counting spectral computed tomography data, the window parameters of the energy bins may be adapted with regard to the specific computed tomography application. For example, the window parameters of the energy bins can be preselected to obtain energy resolved imaging data that allows for optimized material decomposition of the energy resolved imaging, for example for any of the materials selected from the group consisting of iodine, calcium and water. In particular, it can be advantageous to provide three or more energy bins to enable a three-material decomposition. Thereby it is possible to obtain the energy resolved imaging data which is optimized for the task of detecting a perfusion defect or a vessel obstruction.

In embodiments, where the segmentation is generated based on the energy resolved CT imaging data, the segmentation may for example be generated based on the PCCT imaging data.

According to several embodiments, the segmentation is generated by applying a first trained machine learning model, MLM, to first input data comprising the medical imaging data at least partially, for example the energy resolved CT imaging data.

In general terms, a trained MLM may mimic cognitive functions that humans associate with other human minds. In particular, by training based on training data the MLM may be able to adapt to new circumstances and to detect and extrapolate patterns. Another term for a trained MLM is "trained function".

For example, a trained MLM can be trained with energy resolved imaging data as training data, wherein the training data has been annotated with additional training information. The additional training information can comprise the annotation of ground truth information. As a non-limiting list of examples, the ground truth information can comprise a classification information which classifies a target region of the imaging data into one of a plurality of classes, a scoring information, which associates a target region of the image with a score (g.g.a disease score), an image segmentation information, a perfusion information, an indication of the presence or absence an obstruction of a vessel in a target region of the imaging data, or an indication of the presence or absence of a perfusion defect in a target region. The annotation can be performed manually by trained personnel and/or automatically by use or with the aid of a respective annotation software.

In general, parameters of an MLM can be adapted or updated by means of training. In particular, supervised training, semi-supervised training, unsupervised training, reinforcement learning and/or active learning can be used. Furthermore, representation learning, also denoted as feature learning, can be used. In particular, the parameters of the MLMs can be adapted iteratively by several steps of training. In particular, within the training a certain loss function, also denoted as cost function, can be minimized. In particular, within the training of an artificial neural network, ANN, the backpropagation algorithm can be used.

In particular, an MLM can comprise an ANN, a support vector machine, a decision tree and/or a Bayesian network, and/or the MLM can be based on k-means clustering, Q-learning, genetic algorithms and/or association rules. In particular, an ANN can be or comprise a deep neural network, a convolutional neural network or a convolutional deep neural network. Furthermore, an ANN can be an adversarial network, a deep adversarial network and/or a generative adversarial network, GAN.

The first MLM may be a known MLM for medical image segmentation, for example an ANN based on the U-Net, which has been trained in a conventional manner.

According to several embodiments, the obstructing object is detected by applying a second trained MLM to second input data comprising the medical imaging data at least partially, for example the energy resolved CT imaging data.

The second MLM may be a known MLM for medical image segmentation or object detection in medical images, for example an ANN based on the U-Net or an algorithm as proposed by Grenier et al. in the above mentioned publication, which has been trained in a conventional manner.

In some embodiments, the first MLM and the second MLM may also be part of a further MLM. In such embodiments, the obstructing object is detected and the segmentation is generated by applying the trained further MLM to at least a part of the medical imaging data, for example of the energy resolved CT imaging data.

According to several embodiments, the energy resolved CT imaging data comprises contrast enhanced CT, CECT, imaging data, for example including a contrast agent map, in particular an iodine map, and/or a DER map.

Consequently, in such embodiments, the perfusion defect score, in particular the segment perfusion defect scores and/or the size of perfusion defect regions and/or the PBV values, may be determined with an increased accuracy.

According to several embodiments, the perfusion defect score is determined by applying a trained third MLM to third input data comprising the medical imaging data.

The third MLM may for example be based on a known MLM architecture, for example the U-Net, which has been trained in a conventional manner. In particular, the training of the third MLM may be based on a plurality of training datasets, each of them comprising respective training imaging data corresponding to the medical imaging data and a corresponding perfusion defect score as a ground truth label.

Consequently, the trained third MLM can directly predict the perfusion defect score. In such embodiments, carrying out the computer-implemented method according to the invention is particularly efficient from a computational point of view.

For example, an output of the third MLM as a result of applying it to the third input data may comprise a location of the obstructing object as well. In this case, the training data sets may also comprise a corresponding location as a ground truth label.

According to several embodiments, the third input data comprises the segmentation including the hierarchical classification.

In the respective training phase, the training data sets also comprise a corresponding training segmentation as an input to the third MLM. In such embodiments, the training effort for the third MLM may be reduced, since the third MLM is given additional information about the content of the training imaging data. Furthermore, also the accuracy of the trained third MLM's output may be increased.

According to a further aspect of the invention, a data processing system is provided. The data processing system is configured to carry out a computer-implemented method according to the invention.

In the present disclosure, the expressions "data processing system" and "at least one data processing device" may be used interchangeably. A data processing device may in particular be understood as a data processing device, which comprises processing circuitry. The data processing device can therefore in particular process data to perform computing operations. This may also include operations to perform indexed accesses to a data structure, for example a look-up table, LUT, as well as a data processing process implemented in hardware.

In particular, the data processing device may include one or more computers, one or more microcontrollers, and/or one or more integrated circuits, for example, one or more application-specific integrated circuits, ASIC, one or more field-programmable gate arrays, FPGA, and/or one or more systems on a chip, SoC. The data processing device may also include one or more processors, for example one or more microprocessors, one or more central processing units, CPU, one or more graphics processing units, GPU, and/or one or more signal processors, in particular one or more digital signal processors, DSP. The data processing device may also include a physical or a virtual cluster of computers or other of said units.

In various embodiments, the data processing device includes one or more hardware and/or software interfaces and/or one or more memory units.

A memory unit may be implemented as a volatile data memory, for example a dynamic random access memory, DRAM, or a static random access memory, SRAM, or as a non-volatile data memory, for example a read-only memory, ROM, a programmable read-only memory, PROM, an erasable programmable read-only memory, EPROM, an electrically erasable programmable read-only memory, EEPROM, a flash memory or flash EEPROM, a ferroelectric random access memory, FRAM, a magnetoresistive random access memory, MRAM, or a phase-change random access memory, PCRAM.

In particular, the data processing system may comprise

an input data interface, configured to receive medical imaging data depicting a vessel structure in at least one organ and the vessel structure is received, wherein the medical imaging data ,) comprises energy resolved CT imaging data,

- a detection module for detecting a blood stream obstructing object in the vessel structure is based on the medical imaging data; and

- determination module for determining a perfusion defect score for a target region of the at least one organ, whose blood perfusion is potentially affected by the obstructing object, wherein said determining is performed depending on the energy resolved CT imaging data.

According to a further aspect of the invention, a medical imaging system is provided. The medical imaging system comprises a data processing system according to the invention and a CT device, which is configured to generate the medical imaging data depicting the at least one organ of the vessel structure.

In particular, the CT device is configured to generate the energy resolved CT imaging data. For example, the CT device may be a photon counting CT device.

Further implementations of the medical imaging system according to the invention follow directly from the various embodiments of the computer-implemented method according to the invention and vice versa. In particular, individual features and corresponding explanations as well as advantages relating to the various implementations of the computer-implemented method according to the invention can be transferred analogously to corresponding implementations of the medical imaging system according to the invention. In particular, the medical imaging system according to the invention is designed or programmed to carry out the computer-implemented method according to the invention. In particular, the medical imaging system according to the invention carries out the computer-implemented method according to the invention.

According to a further aspect of the invention, a computer program comprising instructions is provided. When the instructions are executed by a data processing system, the instructions cause the data processing system to carry out a computer-implemented method according to the invention.

The instructions may be provided as program code, for example. The program code can for example be provided as binary code or assembler and/or as source code of a programming language, for example C, and/or as program script, for example Python.

According to a further aspect of the invention, a computer-readable storage medium storing a computer program according to the invention is provided.

The computer program and the computer-readable storage medium are respective computer program products comprising the instructions.

Further features and feature combinations of the invention are obtained from the figures and their description as well as the claims. In particular, further implementations of the invention may not necessarily contain all features of one of the claims. Further implementations of the invention may comprise features or combinations of features, which are not recited in the claims.

In the following, the invention will be explained in detail with reference to specific exemplary embodiments and respective schematic drawings. In the drawings, identical or functionally identical elements may be denoted by the same reference signs. The description of identical or functionally identical elements is not necessarily repeated with respect to different figures.

In the figures,

FIG 1    shows an exemplary embodiment of a medical imaging system according to the invention;

FIG 2    shows a schematic flow diagram of an exemplary embodiment of a computer-implemented method for imaging-based characterization of a perfusion defect in a vessel structure for blood supply of at least one organ of a patient according to the invention;

FIG 3    shows schematically a bronchial tree and an obstructing object in a use case for a further exemplary embodiment of a computer-implemented method for imaging-based characterization of a perfusion defect according to the invention;

FIG 4    shows schematically a bronchial tree and an obstructing object in a use case for a further exemplary embodiment of a computer-implemented method for imaging-based characterization of a perfusion defect according to the invention;

FIG 5    shows schematically a transversal sectional view of segmented lungs in a use case for a further exemplary embodiment of a computer-implemented method for imaging-based characterization of a perfusion defect according to the invention;

FIG 6    shows schematically a sagittal sectional view of segmented lungs in a use case for a further exemplary embodiment of a computer-implemented method for imaging-based characterization of a perfusion defect according to the invention;

FIG 7    shows a schematic flow diagram of a further exemplary embodiment of a computer-implemented method for imaging-based characterization according to the invention;

FIG 8    shows a schematic flow diagram of a further exemplary embodiment of a computer-implemented method for imaging-based characterization according to the invention;

FIG 9    shows schematically an exemplary embodiment of an artificial neural network;

FIG 10    shows schematically an exemplary embodiment of a convolutional neural network; and

FIG 11    shows schematically a further exemplary embodiment of a convolutional neural network.

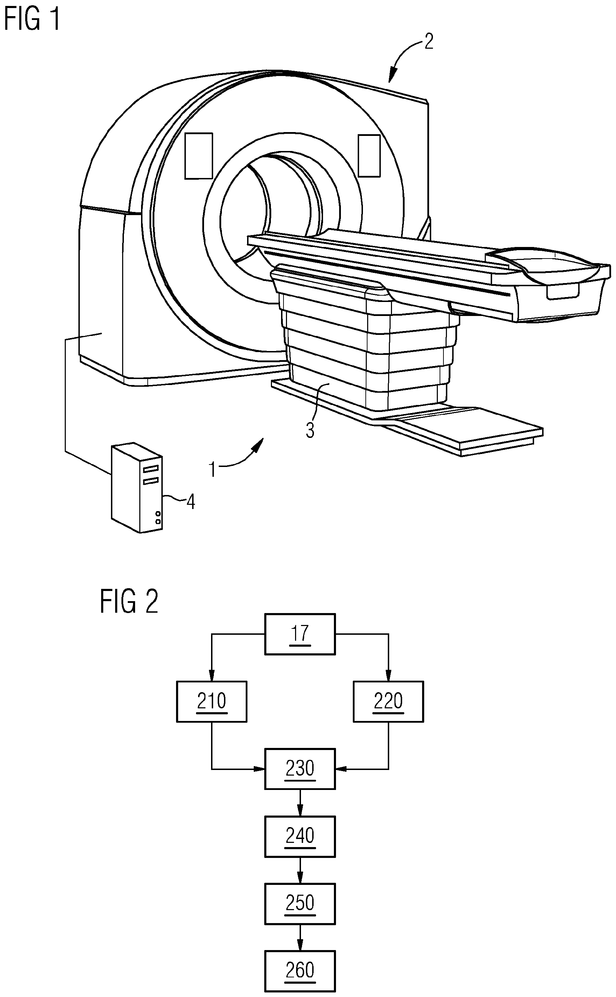

FIG 1 shows an exemplary embodiment of a medical imaging system 1 according to the invention. The medical imaging system 1 comprises a data processing system 4 according to the invention and a CT device 2, which is, in particular, implemented as a photon-counting CT device.

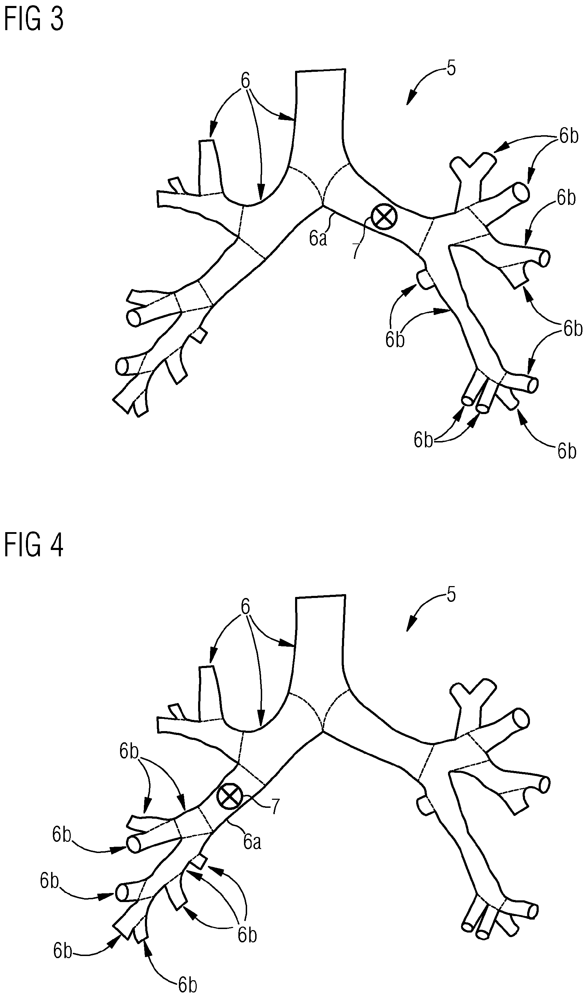

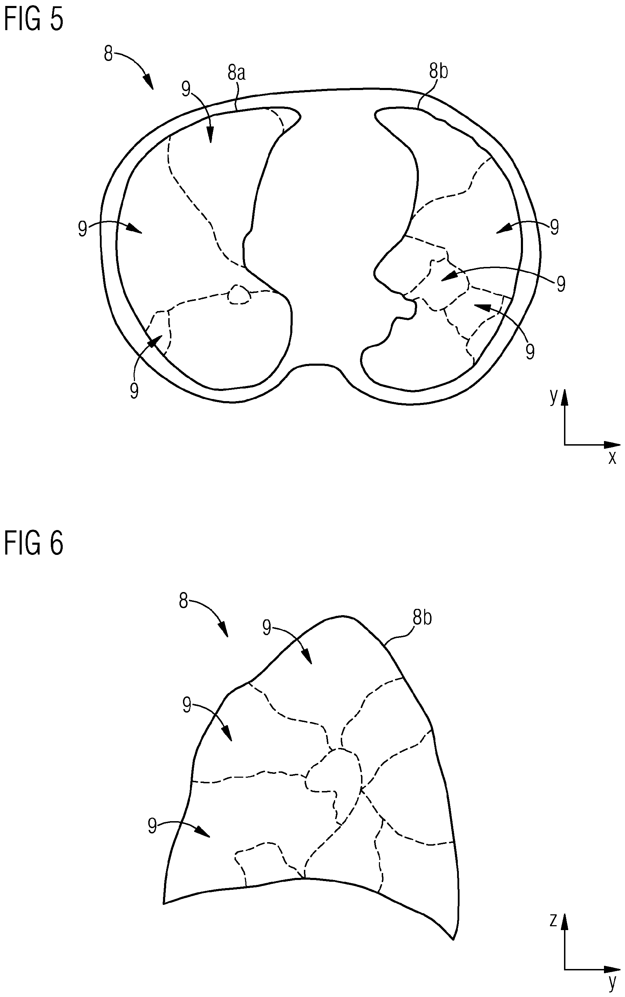

The CT device 2 is configured to generate energy resolved CT imaging data 17, in particular including one or more reconstructed CT image volumes, depicting at least one organ 5, 8 of a patient and a vessel structure for blood supply of at least one organ 5, 8. The at least one organ 5, 8 is, in particular, given by lungs 8 of the patient including a bronchial tree 5, as shown schematically in FIG 3 and FIG 4, and parenchymal tissue of the lungs 8, as shown schematically in FIG 5 and FIG 6. The vessel structure may for example include or correspond to pulmonary arteries.

The data processing system 4 is configured to carry out an exemplary embodiment of a computer-implemented method for imaging-based characterization of a perfusion defect in the vessel structure according to the invention. Schematic flow diagrams of different exemplary embodiments of said computer-implemented method are shown in FIG 2, FIG 7 and FIG 8.

In general, in order to carry out a computer-implemented method according to the invention, the data processing system 4 receives the energy resolved CT imaging data 17 and detects a blood stream obstructing object 7, in particular a blood clot, in the vessel structure based on the energy resolved CT imaging data 17. The data processing system 4 determines a perfusion defect score 16 for a target region 6a, 6b of the at least one organ 5, 8, wherein the blood perfusion of the target region 6a, 6b, is potentially affected by the obstructing object 7, depending on the energy resolved CT imaging data 17.

According to the embodiment of the computer-implemented method depicted in FIG 2, the obstructing object 7 is detected in step 210 using for example a known algorithm, for example an algorithm based on a trained first MLM, for clot detection in pulmonary arteries. Beyond the known method, the detection of the obstructing object 7 may for example be improved by using spectral information of the energy resolved CT imaging data 17, for example a material-labeling to better differentiate between contrast agent, in particular, iodine, and clot material.

In step 220, a segmentation 13 dividing the at least one organ 5, 8 into a plurality of segments 6, 9 is generated based on the medical imaging data 17, wherein the plurality of segments 6, 9 is hierarchically classified according to their blood supply by the vessel structure.

The segmentation may for example be a segmentation of the bronchial tree 5 as shown in FIG 3 and FIG 4. The segmentation may for example be based on the classification by Boyden. The trachea divides into two main bronchi, also known as primary bronchi, at the carina. One main bronchus leads to the right lung 8b, and the other leads to the left lung 8a. Each main bronchus enters the respective lung and further divides. The main bronchi divide into lobar bronchi, also called secondary bronchi. The right lung 8b has three lobes, so the right main bronchus divides into three lobar bronchi, denoted as superior, middle, and inferior lobar bronchi. The left lung 8a has two lobes, so the left main bronchus divides into two lobar bronchi, namely superior and inferior lobar bronchi. The lobar bronchi further divide into segmental bronchi. The segmental bronchi supply specific segments of lung tissue known as bronchopulmonary segments. Each lung has a varying number of bronchopulmonary segments, with the right lung 8b having more bronchopulmonary segments compared to the left lung 8a. The segmental bronchi continue to divide into smaller bronchi known as subsegmental bronchi. These subsegmental bronchi supply even smaller regions within the bronchopulmonary segments.

The bronchial tree 5 is not only responsible for the passage of air but also requires its own blood supply by the pulmonary arteries. The pulmonary arteries branch off from the systemic circulation and supply oxygenated blood to the bronchi, bronchioles, and other lung structures. Each segment 6 of the bronchial tree 5 has its own pulmonary arterial branch and thus, each lung segment 9, as shown FIG 5 and FIG 6, is a portion of lung supplied by its own bronchus and artery. Each segment is functionally and anatomically discrete allowing a single segment to be surgically resected without affecting its neighboring segments. Thus, each segment 6 of the bronchial tree 5 can also be associated to a respective segment 9 of the parenchymal tissue of the lungs 8 and, in particular, an obstruction of the blood flow in a part of the vessel structure supplying a certain segment 6 of the bronchial tree 5 potentially affects the associated blood perfusion of the corresponding segment 9 of the parenchymal tissue of the lungs 8.

The classification of bronchi according to Boyden refers to the standard nomenclature used to describe bronchopulmonary segmental anatomy. The described bronchopulmonary segment model is a preferred segmentation. However, the segmentation may also be based on another segment model, for example of the lung lobes, left and right lung 8b, or other customized lung segment definitions.

In the method of FIG 2, the target region 6a, 6b is determined to comprise one or more target segments 6a, 6b of the plurality of segments 6, 9, whose blood perfusion is potentially affected by the obstructing object 7 according to the hierarchical classification. It is noted that the location of the obstructing object 7 is shown in FIG 3 and FIG 4 in certain segments 6a of the bronchial tree 5 for illustrative reasons. This does not mean that the object 7 is located inside the respective bronchus but rather in the corresponding part of the vessel structure.

As shown in FIG 5 and FIG 6, the parenchymal tissue of the lungs 8 may be segmented based on a known algorithm, for example based on a trained second MLM. For example, the second MLM may be trained on ultra-high-resolution photon-counting CT data that better depicts the intersegmental fissures allowing for a better anatomical delineation of the lung segments.

In step 230, the root segment 6a of the bronchial segments 6, which is the segment 6 corresponding to the location of the obstructing object 7, and/or the corresponding root segment of the parenchymal tissue of the lungs 8 is automatically identified based on the segmentation 13.

Based on the hierarchical anatomical bronchopulmonary segment model, all dependent downstream segments 6b of the bronchial tree 5 and/or all dependent downstream segments of the parenchymal tissue of the lungs 8 are identified in step 240.

In each of the downstream segments of the parenchymal tissue of the lungs 8, the parenchymal perfusion is quantified in step 250 based on spectral perfusion data obtained from the energy resolved CT imaging data 17, for example based on the iodine uptake or dual energy ratio DER. For example, the spectral perfusion data may be normalized based on reference values in an automatically identified reference region, for example the pulmonary trunk, to account for contrast bolus variations in-between different scans and patients. The quantification may for example be done by comparing the iodine concentration, enhancement values or DER between the downstream segments of the parenchymal tissue of the lungs 8 and normal lung segments. The normal lung segments may for example be defined as the perfusion in a normal reference population or as the perfusion in non-affected downstream segments in a given patient.

In step 260, the perfusion defect score 16 is then aggregated from the segment-wise perfusion defect quantification. The perfusion defect score 16 may for example be determined as the number of segments with perfusion defects or significant perfusion defects, a total volume of the perfusion defect regions, a percentage of lung involvement calculated by dividing the volume of the perfusion defects by the total lung volume, et cetera.

The steps 210 to 260 may also be carried out repeatedly for different associated obstructing objects 7, see FIG 3 in comparison to FIG 4, for example. In particular, the associated obstructing objects 7 may be ranked or filtered according to their severeness given by their perfusion defect score 16. Also, in some embodiments, perfusion defects which could not be explained by associated obstructing objects 7 may be marked for further analysis.

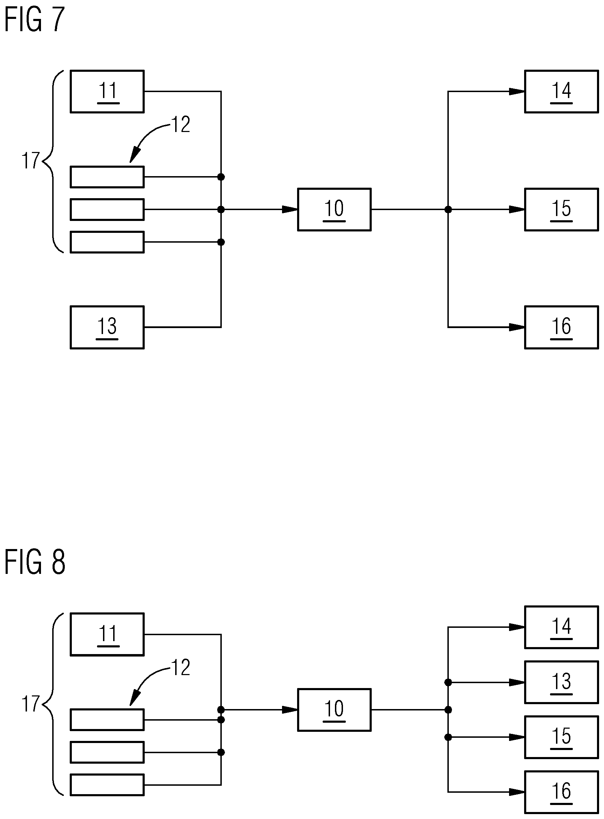

FIG 7 shows a schematic flow diagram of a further exemplary embodiment of a computer-implemented method for imaging-based characterization according to the invention. Therein, the perfusion defect score 16 is determined by applying a trained third MLM 10 to third input data comprising the energy resolved CT imaging data 17. In particular, in the embodiment of FIG 7, the detection of the location 14 of the obstructing object 7 or of multiple such obstructing objects 7 and associated perfusion defect scores 16 is combined intrinsically into the third MLM 10, which may be based on a U-Net or a transformer model, for example.

The third MLM 10 receives as an input, for example, a CECT image volume 11, preferably with ultra-high resolution, and spectral information 12, such as the associated iodine image or DER-image. Optionally, the segmentation 13 with hierarchical labeling may be passed to the third MLM 10 as an additional input to guide the third MLM 10 towards adhering to given lung segments. If the lung segments are not passed as input, the third MLM 10 may for example identify perfusion defect regions not adhering to any prior segment model.

The output of the third MLM 10 includes, for example, the respective locations of the detected obstructing objects 7. Additionally, detection algorithm may also be trained on the spectral information 12 that helps to differentiate blood clot material from iodine, for example. The output of the third MLM 10 also includes the respective perfusion defect scores 16. Optionally, the output of the third MLM 10 includes respective detected regions with perfusion defects and corresponding perfusion defect quantifications for each obstructing objects.

FIG 8 shows a schematic flow diagram of a further exemplary embodiment of a computer-implemented method for imaging-based characterization according to the invention. As explained for FIG 7, the perfusion defect score 16 is determined by applying the trained third MLM 10 to the third input data comprising the energy resolved CT imaging data 17. The input for the third MLM 10 includes, for example, the CECT image volume 11 and the spectral information 12, but not the segmentation 13. Rather, in this embodiment the third MLM is trained on an auxiliary task predict the segmentation 13 as part of its output.

The third MLM 10 for the embodiments of FIG 7 and FIG 8 above are for example trained in a supervised manner based on expert annotations for the output data to be estimated.

In several embodiments of the computer-implemented method according to the invention, clot detection and parenchymal perfusion defect assessment are combined to improve the detection of severe pulmonary embolism thrombi with associated perfusion defects.

In several embodiments, PCCT imaging data is used as a basis leveraging the intrinsic benefits of ultra-high-resolution and spectral image information provided by PCCT scans. This helps to separate pulmonary embolisms that do not have severe impact to the patient from more critical ones. For example, a blood clot may have a hyperdense appearance, which is difficult to differentiate on contrast-enhanced conventional CT images. However, blood clots and iodine have distinct material compositions that can be assessed by spectral PCCT more accurately.

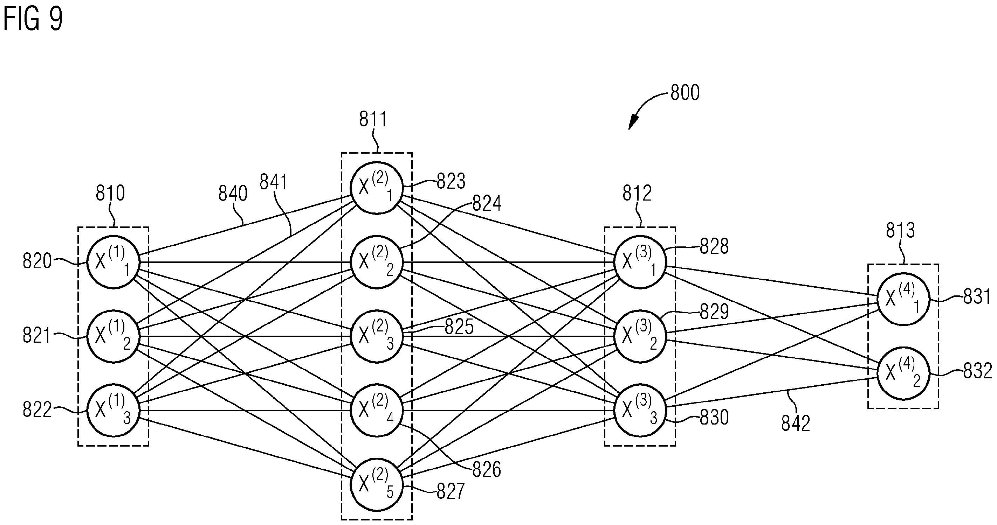

As explained above, several method steps may be carried out using respectively trained MLMs, for example ANNs. FIG 9 displays an embodiment of an ANN 800, which is for example designed as an MLP. The ANN 800 comprises nodes 820, ..., 832 and edges 840, ..., 842, wherein each edge 840, ... , 842 is a directed connection from a first node 820, ... , 832 to a second node 820, ..., 832. In general, the first node 820, ... , 832 and the second node 820, ..., 832 are different nodes 820, ... , 832. It is, however, also possible that the first node 820, ... , 832 and the second node 820, ... , 832 are identical. For example, in FIG 9, the edge 840 is a directed connection from the node 820 to the node 823, and the edge 842 is a directed connection from the node 830 to the node 832. An edge 840, ..., 842 from a first node 820, ..., 832 to a second node 820, ... , 832 is also denoted as ingoing edge for the second node 820, ... , 832 and as outgoing edge for the first node 820, ... , 832.

In this example, the nodes 820, ... , 832 of the artificial neural network 800 can be arranged in layers 810, ... , 813, wherein the layers can comprise an intrinsic order introduced by the edges 840, ... , 842 between the nodes 820, ... , 832. In particular, edges 840, ... , 842 can exist only between neighboring layers of nodes. In the displayed example, there is an input layer 810 comprising only nodes 820, ... , 822 without an incoming edge, an output layer 813 comprising only nodes 831, 832 without outgoing edges, and hidden layers 811, 812 in-between the input layer 810 and the output layer 813. In general, the number of hidden layers 811, 812 can be chosen arbitrarily. In an MLP, this number is at least one. The number of nodes 820, ..., 822 within the input layer 810 usually relates to the number of input values of the artificial neural network 800, and the number of nodes 831, 832 within the output layer 813 usually relates to the number of output values of the artificial neural network 800.

In particular, a real number can be assigned as a value to every node 820, ... , 832 of the artificial neural network 800. Here, $x^{(n)}_i$ denotes the value of the i-th node 820, ... , 832 of the n-th layer 810, ... , 813. The values of the nodes 820, ... , 822 of the input layer 810 are equivalent to the input values of the artificial neural network 800. The values of the nodes 831, 832 of the output layer 813 are equivalent to the output value of the artificial neural network 800. Furthermore, each edge 840, ... , 842 can comprise a weight being a real number. In particular, the weight is a real number within the interval [-1, 1] or within the interval [0, 1]. Here, $w^{(m,n)}_{i,j}$ denotes the weight of the edge between the i-th node 820, ... , 832 of the m-th layer 810, ... , 813 and the j-th node 820, ... , 832 of the n-th layer 810, ... , 813. Furthermore, the abbreviation $w^{(n)}_{i,j}$ is defined for the weight $w^{(n,n+1)}_{i,j}$. In particular, to calculate the output values of the neural network 800, the input values are propagated through the neural network 800. In particular, the values of the nodes 820, ... , 832 of the (n+1)-th layer 810, ..., 813 can be calculated based on the values of the nodes 820, ... , 832 of the n-th layer 810, ..., 813 by

$$x_j^{(n+1)} = f\left(\sum_i x_i^{(n)} \; w_{i,j}^{(n)}\right)$$

Herein, the function f is denoted as transfer function or activation function. Known transfer functions are step functions, the sigmoid functions, for example the logistic function, the generalized logistic function, the hyperbolic tangent, the arctangent function, the error function, the smoothstep function, or rectifier functions. The transfer function is for example used for normalization purposes. In particular, the values are propagated layer-wise through the neural network 800, wherein values of the input layer 810 are given by the input of the neural network 800, wherein values of the first hidden layer 811 can be calculated based on the values of the input layer 810 of the neural network 800, wherein values of the second hidden layer 812 can be calculated based on the values of the first hidden layer 811, and so forth.

In order to set the values $w^{(m,n)}_{i,j}$ for the edges, the neural network 800 has to be trained using training data. In particular, training data comprises training input data and training output data (denoted as $t_i$). For a training step, the neural network 800 is applied to the training input data to generate calculated output data. In particular, the training data and the calculated output data comprise a number of values, said number being equal with the number of nodes of the output layer. In particular, a comparison between the calculated output data and the training data is used to recursively adapt the weights within the neural network 800 (backpropagation algorithm). In particular, the weights are changed according to

$$w_{i,j}^{'(n)} = w_{i,j}^{(n)} - \gamma \, \delta_j^{(n)} \, x_i^{(n)},$$

wherein $\gamma$ is a predefined learning rate, and the numbers $\delta^{(n)}_j$ can be recursively calculated as

$$\delta_j^{(n)} = \left( \sum_k \delta_k^{(n+1)} \, w_{j,k}^{(n+1)} \right) f' \left( x_i^{(n)} w_{i,j}^{(n)} \right)$$

based on $\delta^{(n+1)}_j$, if the (n+1)-th layer is not the output layer 813, and

$$\delta_j^{(n)} = \left( x_j^{(n+1)} - t_j^{(n+1)} \right) \, f' \left( x_i^{(n)} w_{i,j}^{(n)} \right),$$

if the (n+1)-th layer is the output layer 813, wherein f' is the first derivative of the activation function, and $t^{(n+1)}_j$ is the comparison training value for the j-th node of the output layer 813.

The ANN may for example be a convolutional neural network, CNN. A CNN is an ANN that uses a convolution operation instead of general matrix multiplication in at least one of its layers. These layers are denoted as convolutional layers. In particular, a convolutional layer performs a dot product of one or more convolution kernels with the convolutional layer's input data, wherein the entries of the one or more convolution kernel are parameters or weights that may be adapted by training. In particular, one can use the Frobenius inner product and the ReLU activation function. A convolutional neural network can comprise additional layers, for example pooling layers, fully connected layers, and/or normalization layers.

By using convolutional neural networks, the input can be processed in a very efficient way because a convolution operation based on different kernels can extract various image features so that by adapting the weights of the convolution kernel the relevant image features can be found during training. Furthermore, based on the weight-sharing in the convolutional kernels fewer parameters need to be trained, which prevents overfitting in the training phase and allows to have faster training or more layers in the network, improving the performance of the network.

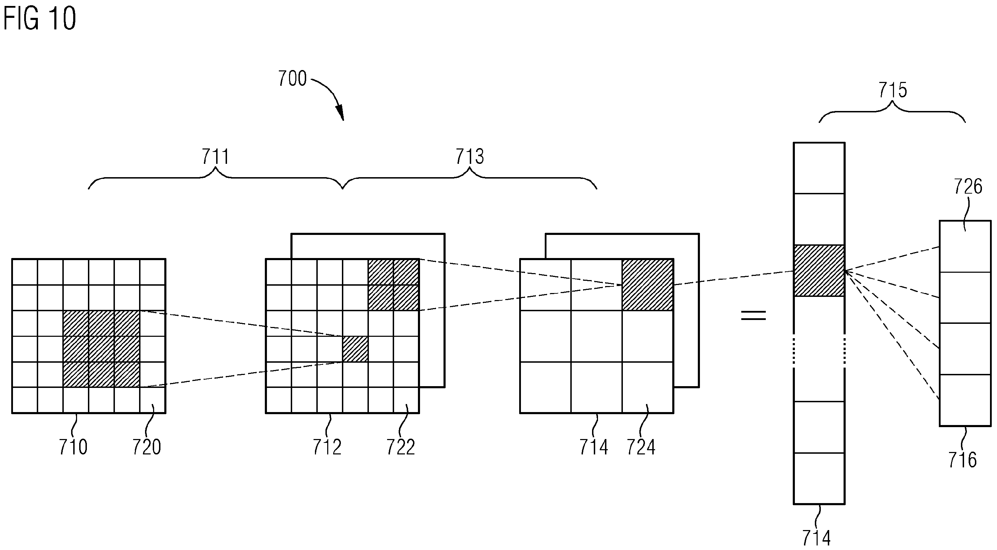

FIG 10 displays an exemplary embodiment of a convolutional neural network 700. In the displayed embodiment, the convolutional neural network 700 comprises an input node layer 710, a convolutional layer 711, a pooling layer 713, a fully connected layer 714 and an output node layer 716, as well as hidden node layers 712, 714. Alternatively, the convolutional neural network 200 can comprise several convolutional layers 711, several pooling layers 713 and/or several fully connected layers 715, as well as other types of layers. The order of the layers can be chosen arbitrarily, usually fully connected layers 715 are used as the last layers before the output layer 716.

In particular, within a convolutional neural network 700 nodes 720, 722, 724 of a node layer 710, 712, 714 can be considered to be arranged as a d-dimensional matrix or as a d-dimensional image. In particular, in the two-dimensional case the value of the node 720, 722, 724 indexed with i and j in the n-th node layer 710, 712, 714 can be denoted as x(n)[i, j]. However, the arrangement of the nodes 720, 722, 724 of one node layer 710, 712, 714 does not have an effect on the calculations executed within the convolutional neural network 700 as such, since these are given solely by the structure and the weights of the edges.

A convolutional layer 711 is a connection layer between an anterior node layer 710 with node values x(n-1) and a posterior node layer 712 with node values x(n). In particular, a convolutional layer 711 is characterized by the structure and the weights of the incoming edges forming a convolution operation based on a certain number of kernels. In particular, the structure and the weights of the edges of the convolutional layer 711 are chosen such that the values x(n) of the nodes 722 of the posterior node layer 712 are calculated as a convolution x(n) = K * x(n-1) based on the values x(n-1) of the nodes 720 anterior node layer 710, where the convolution * is defined in the two-dimensional case as

$$x^{(n)}[i,j] = \left( K * x^{(n-1)} \right)[i,j] = \sum_{i'} \sum_{j'} K[i',j'] \cdot x^{(n-1)}[i - i', j - j']$$

Herein, the kernel K is a d-dimensional matrix, in the present example a two-dimensional matrix, which is usually small compared to the number of nodes 720, 722, for example a 3x3 matrix, or a 5x5 matrix. In particular, this implies that the weights of the edges in the convolution layer 711 are not independent, but chosen such that they produce said convolution equation. In particular, for a kernel being a 3x3 matrix, there are only 9 independent weights, each entry of the kernel matrix corresponding to one independent weight, irrespectively of the number of nodes 720, 722 in the anterior node layer 710 and the posterior node layer 712.

In general, convolutional neural networks 700 use node layers 710, 712, 714 with a plurality of channels, in particular, due to the use of a plurality of kernels in convolutional layers 711. In those cases, the node layers can be considered as (d+1)-dimensional matrices, the first dimension indexing the channels. The action of a convolutional layer 711 is then in a two-dimensional example defined as

$$x_b^{(n)}[i,j] = \sum_a (K_{a,b} * x_a^{(n-1)}[i,j]) = \sum_a \sum_{i'} \sum_{j'} K_{a,b}[i',j'] \cdot x_a^{(n-1)}[i-i',j-j'],$$

wherein $x_a^{(n)}$ corresponds to the a-th channel of the anterior node layer 710, $x_b^{(n)}$ corresponds to the b-th channel of the posterior node layer 712 and $K_{a,b}$ corresponds to one of the kernels. If a convolutional layer 711 acts on an anterior node layer 710 with A channels and outputs a posterior node layer 712 with B channels, there are A·B independent d-dimensional kernels $K_{a,b}$.

In general, in convolutional neural networks 700 activation functions may be used. In this embodiment, ReLU (rectified linear unit) is used, with R(z) = max(0, z), so that the action of the convolutional layer 711 in the two-dimensional example is

$$x_b^{(n)}[i,j] = R\left(\sum_a (K_{a,b} * x_a^{(n-1)}[i,j])\right) = R\left(\sum_a \sum_{i'} \sum_{j'} K_{a,b}[i',j'] \cdot x_a^{(n-1)}[i-i',j-j']\right)$$

It is also possible to use other activation functions, for example ELU (exponential linear unit), LeakyReLU, Sigmoid, Tanh or Softmax.

In the displayed embodiment, the input layer 710 comprises 36 nodes 720, arranged as a two-dimensional 6x6 matrix. The first hidden node layer 712 comprises 72 nodes 722, arranged as two two-dimensional 6x6 matrices, each of the two matrices being the result of a convolution of the values of the input layer with a 3x3 kernel within the convolutional layer 711. Equivalently, the nodes 722 of the first hidden node layer 712 can be interpreted as arranged as a three-dimensional 2x6x6 matrix, wherein the first dimension correspond to the channel dimension.

An advantage of using convolutional layers 711 is that spatially local correlation of the input data can exploited by enforcing a local connectivity pattern between nodes of adjacent layers, in particular by each node being connected to only a small region of the nodes of the preceding layer.

A pooling layer 713 is a connection layer between an anterior node layer 712 with node values x(n-1) and a posterior node layer 714 with node values x(n). In particular, a pooling layer 713 can be characterized by the structure and the weights of the edges and the activation function forming a pooling operation based on a non-linear pooling function f. For example, in the two-dimensional case the values x(n) of the nodes 724 of the posterior node layer 714 can be calculated based on the values x(n-1) of the nodes 722 of the anterior node layer 712 as

$$x_b^{(n)}[i,j] = f\left(x_b^{(n-1)}[id_1, jd_2], \dots, \quad x_b^{(n-1)}[(i+1)d_1 - 1, (j+1)d_2 - 1]\right)$$

In other words, by using a pooling layer 713, the number of nodes 722, 724 can be reduced by re-placing a number d1 d2 of neighboring nodes 722 in the anterior node layer 712 with a single node 722 in the posterior node layer 714 being calculated as a function of the values of said number of neighboring nodes. In particular, the pooling function f can be the max-function, the average or the L2-Norm. In particular, for a pooling layer 713 the weights of the incoming edges are fixed and are not modified by training.

The advantage of using a pooling layer 713 is that the number of nodes 722, 724 and the number of parameters is reduced. This leads to the amount of computation in the network being reduced and to a control of overfitting.

In the displayed embodiment, the pooling layer 713 is a max-pooling layer, replacing four neighboring nodes with only one node, the value being the maximum of the values of the four neighboring nodes. The max-pooling is applied to each d-dimensional matrix of the previous layer. In this embodiment, the max-pooling is applied to each of the two two-dimensional matrices, reducing the number of nodes from 72 to 18.

In general, the last layers of a convolutional neural network 700 may be fully connected layers 715. A fully connected layer 715 is a connection layer between an anterior node layer 714 and a posterior node layer 716. A fully connected layer 713 can be characterized by the fact that a majority, in particular, all edges between nodes 714 of the anterior node layer 714 and the nodes 716 of the posterior node layer are present, and wherein the weight of each of these edges can be adjusted individually.

In this embodiment, the nodes 724 of the anterior node layer 714 of the fully connected layer 715 are displayed both as two-dimensional matrices, and additionally as non-related nodes, indicated as a line of nodes, wherein the number of nodes was reduced for a better presentability. This operation is also denoted as flattening. In this embodiment, the number of nodes 726 in the posterior node layer 716 of the fully connected layer 715 smaller than the number of nodes 724 in the anterior node layer 714. Alternatively, the number of nodes 726 can be equal or larger.

Furthermore, in this embodiment the Softmax activation function is used within the fully connected layer 715. By applying the Softmax function, the sum the values of all nodes 726 of the output layer 716 is 1, and all values of all nodes 726 of the output layer 716 are real numbers between 0 and 1. In particular, if using the convolutional neural network 700 for categorizing input data, the values of the output layer 716 can be interpreted as the probability of the input data falling into one of the different categories.

In particular, convolutional neural networks 700 can be trained based on the backpropagation algorithm. For preventing overfitting, methods of regularization can be used, for example dropout of nodes 720, ... , 724, stochastic pooling, use of artificial data, weight decay based on the L1 or the L2 norm, or max norm constraints.

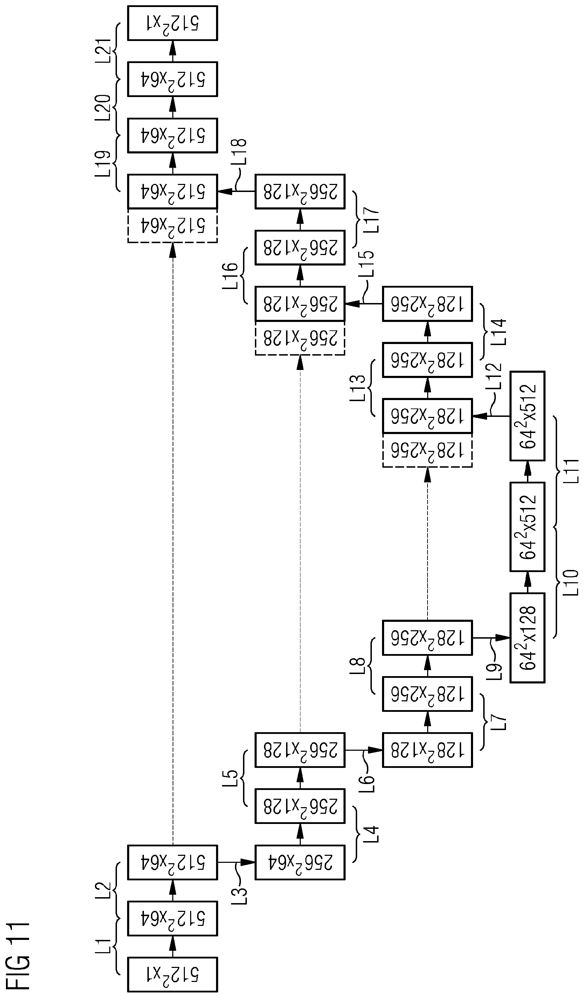

In the example of FIG 11, the CNN has a U-Net structure. In the displayed example, the input data to the CNN is a two-dimensional medical image comprising 512x512 pixels, every pixel comprising one intensity value. The CNN comprises convolutional layers indicated by solid, horizontal arrows, pooling layers indicating by solid arrows pointing down, and upsampling layers indicated by solid arrows pointing up. The number of the respective nodes is indicated within the boxes. Within the U-Net structure first the input images are downsampled, in particular by decreasing the size of the images and increasing the number of channels. Afterwards they are upsampled, in particular by increasing the size of the images and decreasing the number of channels, to generate a transformed image.

All except the last convolutional layers L1, L2, L4, L5, L.7, L8, L10, L11, L13, L14, L16, L17, L19, L20 use 3x3 kernels with a padding of 1, the ReLU activation function, and a number of filters or convolutional kernels that matches the number of channels of the respective node layers as indicated in FIG 11. The last convolutional layer uses a 1x1 kernel with no padding and the ReLU activation function.

The pooling layers L3, L6, L9 are max-pooling layers, replacing four neighboring nodes with only one node, the value being the maximum of the values of the four neighboring nodes. The upsampling layers L12, L15, L18 are transposed convolution layers with 3x3 kernels and stride 2, which effectively quadruple the number of nodes. The dashed horizontal arrows correspond to concatenation operations, where the output of a convolutional layer L2, L5, L8 of the downsampling branch of the U-Net structure is used as additional inputs for a convolutional layer L13, L16, L19 of the upsampling branch of the U-Net structure. This additional input data is treated as additional channels in the input node layer for the convolutional layer L13, L16, L19 of the upsampling branch.

For training the CNN, a database of 500 first medical images was used, wherein the respective segmentation mask was created based on annotations of expert radiologists. In particular, the experts determined for each of the 500 first medical images a segmentation mask for a structure of interest, where a value of 1 was assigned to pixels corresponding to the structure of interest, and a value of 0 was assigned to pixels not corresponding to the structure of interest. The database was split into training data (320 datasets), validation data (80 datasets) and test data (100 datasets). For training the CNN, the backpropagation algorithm was used based on a binary cross-entropy cost function

$$L(x,y) = \sum_i \sum_j BCE(y[i,j], M(x)[i,j])$$

with

$$BCE(a,b) := -a \log(b)\,(b) - (1-a)\log(1-b),$$

wherein x denotes a first medical image, y determines the corresponding segmentation mask created by the expert radiologist, and M(x) denotes the result of applying the CNN to the first input medical image x. Alternatively, one could use other cost functions like weighted binary cross entropy, Focal Loss or Dice Loss.

Based on the validation set of 80 datasets and the corresponding annotations, the best performing machine learning model out of several machine learning models (with different hyperparameters, for example number of layers, size and number of kernels, padding et cetera) was selected. The specificity and the sensitivity were determined based on the test set comprising 100 datasets and the corresponding annotations.

Independent of the grammatical term usage, individuals with male, female or other gender identities are included within the term "patient".

**Claims**

1. Computer-implemented method for imaging-based characterization of a perfusion defect in a vessel structure for blood supply of at least one organ (5, 8) of a patient, wherein

   - medical imaging data (11, 12, 17) depicting the at least one organ (5, 8) and the vessel structure is received, wherein the medical imaging data (11, 12, 17) comprises energy resolved CT imaging data (11, 12, 17);
   - a blood stream obstructing object (7) in the vessel structure is detected based on the medical imaging data (11, 12, 17); and
   - a perfusion defect score (16) for a target region (6a, 6b) of the at least one organ (5, 8), whose blood perfusion is potentially affected by the obstructing object (7), is determined depending on the energy resolved CT imaging data (11, 12, 17).

2. Computer-implemented method according to claim 1, wherein

   - a segmentation (13) dividing the at least one organ (5, 8) into a plurality of segments (6, 9) is generated based on the medical imaging data (11, 12, 17), wherein the plurality of segments (6, 9) is hierarchically classified according to their blood supply by the vessel structure; and
   - the target region (6a, 6b) is determined to comprise one or more target segments (6a, 6b) of the plurality of segments (6, 9), whose blood perfusion is potentially affected by the obstructing object (7) according to the hierarchical classification.

3. Computer-implemented method according claim 2, wherein, for each of the one or more target segments (6a, 6b), a respective segment perfusion defect score is determined based on the energy resolved CT imaging data (11, 12, 17) and the perfusion defect score (16) is determined depending on the segment perfusion defect scores.

4. Computer-implemented method according to claim 3, wherein, for each of the one or more target segments (6a, 6b), a size of a perfusion defect region (15) in the respective target segment (6a, 6b) is determined based on the energy resolved CT imaging data (11, 12, 17) and the respective segment perfusion defect score is determined depending on the size of the perfusion defect region (15).

5. Computer-implemented method according to one of claims 2 to 4, wherein the segmentation (13) is generated by applying a first trained machine learning model, MLM, to first input data comprising the medical imaging data (11, 12, 17) at least partially.

6. Computer-implemented method according to one of the preceding claims, wherein at least one perfusion blood volume, PBV, value for the target region (6a, 6b) is determined depending on the energy resolved CT imaging data (11, 12, 17) and the perfusion defect score is determined depending on the at least one PBV value.

7. Computer-implemented method according to claim 6 and one of claims 2 to 5, wherein the at least one PBV value comprises a respective segment PBV value for the one or more target segments (6a, 6b).

8. Computer-implemented method according to one of the preceding claims, wherein the medical imaging data (11, 12, 17) comprises photon-counting CT, PCCT, imaging data and the obstructing object (7) is detected based on the PCCT imaging data (11, 12, 17).

9. Computer-implemented method according to one of the preceding claims, wherein the obstructing object (7) is detected by applying a second trained MLM to second input data comprising the medical imaging data (11, 12, 17) at least partially.

10. Computer-implemented method according to one of the preceding claims, wherein the energy resolved CT imaging data (11, 12, 17) comprises contrast enhanced CT, CECT, imaging data (12).

11. Computer-implemented method according to one of the preceding claims, wherein the perfusion defect score (16) is determined by applying a trained third MLM (10) to third input data (11, 12, 13) comprising the medical imaging data (11, 12, 17).

12. Computer-implemented method according to one of the preceding claims, wherein the at least one organ (5, 8) comprises the lungs of the patient.

13. Data processing system (4) configured to carry out a computer-implemented method according to one of the preceding claims.

14. Medical imaging system comprising a data processing system (4) according to claim 13 and a CT device (2), which is configured to generate the medical imaging data (11, 12, 17) depicting the at least one organ (5, 8) and the vessel structure.

15. Computer program product comprising instructions which, when executed by a data processing system (4), cause the data processing system (4) to carry out a computer-implemented method according to one of claims 1 to 12.

FIG 1

FIG 2

FIG 3

FIG 4

# FIG 5

# FIG 6

FIG 7

FIG 8

FIG 9

EP 4 715 730 A1

FIG 10

FIG 11

| Europäisches Patentamt European Patent Office Office européen des brevets | **EUROPEAN SEARCH REPORT** | Application Number EP 24 20 1811 |
|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | HU H. ET AL: "Evaluation of CT angiography obstruction score and pulmonary perfusion defect score using the third-generation dual-source CT for pulmonary embolism", CLINICAL RADIOLOGY, [Online] vol. 78, no. 9, 1 September 2023 (2023-09-01), pages e627-e634, XP093255163, AMSTERDAM, NL ISSN: 0009-9260, DOI: 10.1016/j.crad.2023.04.017 Retrieved from the Internet: URL:https://pdf.sciencedirectassets.com/272460/1-s2.0-S0009926023X00087/1-s2.0-S0009926023002167/main.pdf?hash=a4e4ebc45c46ef4602941de2a87f3220ee277bd90b6c3de1f79dfcc72e358657&host=68042c943591013ac2b2430a89b270f6af2c76d8dfd086a07176afe7c76c2c61&pii=S0009926023002167&tid=spdf-171ce3eb-001f-4343-9e97-078> [retrieved on 2025-03-03] | 1,6-15 | INV. G06T7/00 G06T7/11 |
| A | * page e628, column 1, paragraph 1 - page e630, column 2, paragraph 1 * ----- -/-- | 2-5 | TECHNICAL FIELDS SEARCHED (IPC) G06T A61B |

The present search report has been drawn up for all claims

| Place of search | Date of completion of the search | Examiner |
|---|---|---|
| Munich | 3 March 2025 | Turina, Andreas |

EPO FORM 1503 03.82 (P04C01)

Europäisches
Patentamt

European
Patent Office

Office européen
des brevets

## EUROPEAN SEARCH REPORT

**Application Number**

EP 24 20 1811

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | HAGEN FLORIAN ET AL: "Improved detection of small pulmonary embolism on unenhanced computed tomography using an artificial intelligence-based algorithm - a single centre retrospective study", THE INTERNATIONAL JOURNAL OF CARDIOVASCULAR IMAGING, SPRINGER NETHERLANDS, DORDRECHT, vol. 40, no. 11, 28 August 2024 (2024-08-28), pages 2293-2304, XP037958112, DOI: 10.1007/S10554-024-03222-8 [retrieved on 2024-08-28] | 1,6-15 | |
| A | * abstract * * page 2295, right column, section 'Image analysis on unenhanced chest CT using the AI-based algorithm' * * page 2294, column 1, section 'Introduction' * | 2-5 | |
| A | HEJIE CUI ET AL: "Pulmonary Vessel Segmentation based on Orthogonal Fused U-Net++ of Chest CT Images", ARXIV.ORG, CORNELL UNIVERSITY LIBRARY, 201 OLIN LIBRARY CORNELL UNIVERSITY ITHACA, NY 14853, 3 July 2021 (2021-07-03), XP091007301, DOI: 10.1007/978-3-030-32226-7_33 * abstract * * page 1, section 1 - page 4, paragraph 1 * | 1-15 | **TECHNICAL FIELDS SEARCHED (IPC)** |

The present search report has been drawn up for all claims

| Place of search | Date of completion of the search | Examiner |
|---|---|---|
| Munich | 3 March 2025 | Turina, Andreas |

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **P. A. GRENIER et al.** Deep Learning-Based Algorithm for Automatic Detection of Pulmonary Embolism in Chest CT Angiograms. *Diagnostics (Basel)*, 2023, vol. 13 (7)
- **E. A. BOYDEN**. Segmental Anatomy of the Lungs. A Study of the Patterns of the Segmental Bronchi and Related Pulmonary Vessels. The Blakiston Division, McGraw-Hill Book Company, Inc, 1955
- **H. KOIKE**. Quantification of lung perfusion blood volume (lung PBV) by dual-energy CT in patients with chronic thromboembolic pulmonary hypertension (CTEPH) before and after balloon pulmonary angioplasty (BPA): Preliminary results.. *Eur. J. Radiol.*, 2016, vol. 85 (9), 1607
- **O. RONNEBERGER et al.** U-Net: Convolutional Networks for Biomedical Image Segmentation. *arXiv: 1505.04597*